# EUROPEAN PATENT APPLICATION

(11) **EP 1 714 978 A1**
(43) Date of publication of application: **25.10.2006**
(21) Application number: 05008504.2
(22) Date of filing: 19.04.2005
(51) Int. Cl.: C07K 14/47, C07K 7/08, A61K 38/10, A61K 38/17, G01N 33/68, A61P 35/00

(54) **Peptides useful for diagnosis and therapy of tumors**

(71) Applicant: Deutsches Krebsforschungszentrum Stiftung des öffentlichen Rechts, 69120 Heidelberg (DE); Ruprecht-Karls-Universität Heidelberg, 69120 Heidelberg (DE)
(72) Inventor: Zitzmann, Sabine, 69214 Eppelheim (DE); Mier, Walter, 64625 Bensheim (DE); Krämer, Susanne, 68782 Brühl (DE); Haberkorn, Uwe, 68723 Schwetzingen (DE)
(74) Representative: Schüssler, Andrea

(57) **Abstract**

Described are peptides having the amino acid motif EDYELMDLLAYL and related motifs which are capable of specifically binding to tumor cells. The present invention also relates to various diagnostic and therapeutic uses of peptides and (recombinant) proteins containing said motifs and (recombinant) RNA, (recombinant) DNA and recombinant viruses encoding peptides or proteins containing said motifs.

### Annex to the application documents - subsequently filed sequences listing

SEQUENCE LISTING

<110>  Deutsches Krebsforschungszentrum Stiftung des öffentlichen
       Rechts

<120>  Peptides useful for diagnosis and therapy of tumors

<130>  K 3224EP

<140>  EP 05008504
<141>  2005-04-19

<160>  1

<170>  PatentIn version 3.2

<210>  1
<211>  12
<212>  PRT
<213>  artificial

<220>
<223>  Frop-1 peptide

<400>  1

Glu Asp Tyr Glu Leu Met Asp Leu Leu Ala Tyr Leu
1               5                   10

## Description

The present invention relates to peptides having the amino acid sequence motif EDYELMDLLAYL and related motifs which are capable of specifically binding to tumor cells. The present invention also relates to various diagnostic and therapeutic uses of peptides and (recombinant) proteins containing said motifs and (recombinant) RNA, (recombinant) DNA and recombinant viruses encoding peptides or proteins containing said motifs.

Unfortunately, when imaging or treating tumors, the compounds used (e.g., some tracers, chemotherapeutic agents or radionuclides) often show unspecific distribution at non-tumor sites such as inflammation. However, e.g., for diagnosis of a tumor and potential metastasis specific imaging only of the tumor/metastases is desirable and a prerequisite for selective medical intervention. Thus, the coupling of a tumor specific marker to a tracer is required, otherwise tumor imaging cannot be achieved.

There are several routes for tumor therapy depending on the kind of tumor and its localisation, e.g., surgical excision of the tumor, radiation therapy or chemotherapy. In case of tumor therapy, the unspecific distribution of the therapeutic agent, e.g., chemotherapeutic drug, in the organism requires the application of high amounts of the drug in order to achieve a sufficiently high concentration within the organ to be treated resulting in severe side effects and damages of healthy tissue. In addition often the concentration of the therapeutic molecule within the desired organ is quite low, thus, not all tumor cells are killed and an effective treatment is prevented. This applies for chemotherapeutic drugs as well as for radionuclides. For example, if a thyroid tumor still accumulates iodine, radioactive iodine can be used for diagnostic imaging and therapy. However, if the tumor no longer accumulates iodine, only surgical excision can be applied. External radiation is only available for palliative purposes.

In order to overcome the above discussed problems, for tumor imaging several alternative approaches were tried. Although for mammary carcinomas, thyroid carcinoma, cervix carcinoma or prostate carcinoma being in a progressive stage there are diagnostic methods available (using FDG-PET, fluoroethylcholine-PET or choline-PET), therapeutic approaches are quite limited. For therapy of tumors direct delivery of radionuclides or chemotherapeutic drugs to the tumor/metastases is desired in order to achieve high local doses that are required for successful therapy, e.g., by use of tumor specific (labelled) antibodies (e.g., anti-Her2/Neu for mamma carcinoma or Rituximab for lymphoma). So far, antibodies against follicular thyroid carcinoma are not available. There are reports of use of unspecific tracers like MIBI or use of tracers allowing to record tumor metabolism (e.g., fluorodeoxyglucose) or use of peptides specific for the expression of tumor receptors (e.g., Octreotide for recording somatostatin receptors or cholecystokinin-B/gastrin receptor binding peptides for recording medullary cancer of the thyroid gland).

There are also some reports as regards the possible usefulness of somatostatin receptor ligands for therapy of thyroid tumors, since a subset of there tumors (whose which are not concentrating iodine) are resistant to radiation therapy and is has to be evaluated whether sufficient doses of radiation actually can be achieved in the tumor. Cholecystokinin-B/gastrin receptors are only expressed on medullary cancer of the thyroid gland, but not on a papillary or follicular cancer of the thyroid gland. Unfortunately, PET using fluorodeoxyglucose shows in some kinds of tumors (e.g., prostate carcinoma) only a very low accumulation and, thus its diagnostic application for such kind of tumors is limited. Unfortunately, the approaches for tumor imaging discussed above exhibit a variety of additional disadvantages, e.g., undesired immune responses of the patients when using humanized antibodies, slow accumulation of the antibodies due to their large size etc. Octreotide is only useful for tumors showing expression of SSTR (e.g., neuroendocrine tumors, meningiomas, small cell lung carcinomas). For mamma, cervix and prostate carcinomas, there are, so far, no systemic therapies with radionuclides available, i.e., a sufficient tumor specific accumulation of the therapeutic agent (radionuclide or chemotherapeutioc drug) is not possible.

Therefore, it is the object of the present invention to provide means for the efficient, reliable and specific imaging of tumors and metastasis as well as therapy which overcomes the disadvantages of the diagnostic and therapeutic approaches presently used.

According to the present invention this is achieved by the subject matters defined in the claims.

Phage display peptide libraries, that express 12 amino acid long linear peptides, have been used to allow the selection of peptide sequences with desired binding specificities. In this phage system, 10⁹ different peptides motifs are expressed on the phage surface as peptides fused to phage surface proteins. The desired peptide is selected on its binding to the target cell. By using this approach, a novel peptide, FROP-1 (aa sequence EDYELMDLLAYL) was isolated showing specific binding to tumor cells, i.e., it accumulates in vivo at/in tumors. Therefore, it is generally possible to visualize tumors and metastasis through use of the peptide of the present invention when coupled to a compound that is detectable by imaging techniques. In fact, attaching a DOTA as a chelator, which allows labeling of the peptide with various radioisotopes as an imaging and therapeutic tracer, has shown even improved binding properties.
Moreover, the peptide of the invention when coupled to a compound exerting a therapeutic effect, e.g., a cytotoxin, can be used for tumor therapy. The peptide of the invention also allows to evaluate the dynamics of tumor progression in a patient and to monitor the effect of therapy.

Therapy/imaging of tumors or metastasis by use of the peptide of the invention exhibits several advantages:
(a) large amounts of the peptide can be easily prepared;
(b) it does not elicit undesired immune responses;
(c) it can be used in combination with already approved drugs and compounds;
(d) the peptide targets the tumor tissue specifically without affecting normal tissue. Thus, in therapy side effects of therapeutic compounds coupled to the peptide are minimized. Accordingly, high concentrations of the therapeutic compound at the site of the tumor can be achieved (resulting in efficient killing of the tumor) and at the same time the poisoning of the normal tissue can be reduced; and
(e) as regards the diagnostic aspect, the location and size of the tumor/metastasis can be exactly determined, thus, allowing a more precise planning and carrying out of therapy.

Accordingly, the present invention relates to a FROP-1 peptide capable of specifically binding to tumor cells, wherein said peptide comprises the amino acid sequence EDYELMDLLAYL.

The term "specifically binding to tumor cells" as used herein means that, on one hand, the peptide binds to tumor cells with a stability that is sufficient for therapeutic or diagnostic purposes and, on the other hand, it does not bind to non-tumor cells or binds to the non-tumor cells to a substantially lesser extent compared to tumor cells.

The present invention also relates to a peptide with selective binding to tumor cells, wherein said peptide comprises
(a) a peptide having an amino acid sequence which shows at least 60%, preferably at least 80%, more preferably 85% and even more preferably 90% identity to the amino acid sequence EDYELMDLLAYL without losing its capability of specifically binding to a tumor; or
(b) a peptide which is a fragment of the peptides discussed above without losing its capability of specifically binding to a tumor.

Preferably, amino acid differences are due to one or more conservative amino acid substitutions. The term "conservative amino acid substitutions" involves replacement of the aliphatic or hydrophobic amino acids Ala, Val, Leu and Ile; replacement of the hydroxyl residues Ser and Thr; replacement of the acidic residues Asp and Glu; replacement of the amide residues Asn and Gln, replacement of the basic residues Lys, Arg, and His; replacement of the aromatic residues Phe, Tyr, and Trp, and replacement of the small-sized amino acids Ala, Ser, Thr, Met, and Gly.

For the generation of peptides showing a particular degree of identity to the peptide FROP-1, e.g., genetic engineering can be used to introduce amino acid changes at specific positions of a cloned DNA sequence to identify regions critical for peptide function. For example, site directed mutagenesis or alanine-scanning mutagenesis (introduction of single alanine mutations at every residue in the molecule) can be used. (Cunningham and Wells, Science 244 (1989), 1081-1085) The resulting mutant molecules can then be tested for biological activity using the assay of Example 2.

Preferably, the peptides of the invention are characterized by not more than 8 aa substitutions, more preferably by not more than 6 aa substitutions and, even more preferably, by not more than 4 aa substitutions.

In the fragment of the peptide of the invention at least 3 contiguous aa, preferably at least 5 contiguous aa, more preferably at least contiguous 7 aa and even more preferably at least 9 contiguous aa of the amino acid sequence EDYELMDLLAYL are left. The fragment is still capable of specifically binding to a tumor cell. For evaluating whether a particular fragment (or derivative of the peptide FROP-1 characterized by substitutions of conservative amino acids) is still capable of specific binding of tumor cells the assays described in the Examples may be used.

A further embodiment of the invention relates to peptides described above which are operatively attached to a therapeutic agent capable of exerting a therapeutic effect on a tumor. Examples of therapeutic agents useful for the present invention include an anticellular agent, chemotherapeutic agent, a radioisotope, a chelator for the binding of certain radioisotopes or a cytotoxin. Preferred cytotoxins are an A chain toxin, a ribosome inactivating protein, α-sarcin, aspergillin, restrictotin, diphtheria toxin, Pseudomonas exotoxin, a bacterial endotoxin or the lipid A moiety of a bacterial endotoxin. Further suitable proteins for tumor therapy are endostatin (for inhibition of tumor growth) or recombinant chimeric toxin PE37/transforming growth factor alpha (TGF-alpha) (for cytotoxicity to tumor cells).

The present invention also relates to polynucleotides encoding a peptide of the invention, preferably fused to secretory leader sequences, as well as expression vectors which are capable of expressing the peptide of the invention and which can be used for gene therapy. Preferred recombinant vectors useful for gene therapy are viral vectors, e.g. adenovirus, herpes virus, vaccinia, adeno associated virus (AAV) or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene theapy are described in the literature and are known to the persons skilled in the art; see, e.g., WO 94/29469 or WO 97/00957.

The present invention also relates to host cells transformed or transfected with an expression vector of the invention. Suitable host cells include prokaryotes, yeast and higher eukaryotic cells. Preferably, the host cells employed are E. coli, yeast or a mammalian cell line such as COS or CHO or primary mammalian cells such as macrophages or dendritic cells. Supernatants from suitable host/vector systems which secrete a recombinant peptide or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant (poly)peptide.

The present invention also relates to antibodies specifically binding to a peptide of the invention. The term "antibody", preferably, relates to distinct monoclonal antibody preparations. Monoclonal antibodies are made against a peptide of the invention or a fragment thereof as an antigen by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to proteins. Fab and F(ab')2 fragments lack the Fc fragment of intact antibodies, clear more rapidly from the circulation, and may have less non-specific tissue binding than an intact antibody. (Wahl et al., J. Nucl. Med. 24:316-325 (1983).) Thus, these fragments are preferred, as well as the products of a FAB or other immunoglobulin expression library. Moreover, antibodies of the present invention include chimeric, single chain, and humanized antibodies. The target cellular component in biological fluids or tissues, i.e. the peptide of the invention bound to a tumor, may be detected directly in situ or it may be isolated from other cell components by common methods known to those skilled in the art before contacting with a probe. Detection methods include Northern blot analysis, RNase protection, in situ methods, PCR, LCR, immunoassays and other detection assays that are known to those skilled in the art.

In a further embodiment, the present invention relates to a pharmaceutical composition containing a peptide, polynucleotide or expression vector of the invention. For administration the compounds of the pharmaceutical composition are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperitoneal, subcutaneous, intramuscular, topical or intradermal administration. The route of administration, of course, depends on the nature and location of the tumor and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the tumor, general health and other drugs being administered concurrently.

The present invention also relates to a diagnostic composition containing a peptide or antibody of the invention as well as diagnostic kits comprising a peptide, fusion protein and/or antibody of the invention and a detection agent. Such kits typically comprise two or more components necessary for performing a diagnostic assay. Components may be compounds, reagents, containers and/or equipment. For example, one container within a kit may contain a monoclonal antibody or fragment thereof that specifically binds to a peptide of the invention. Such antibodies or fragments may be provided attached to a support material. The solid support may be any material known to those of ordinary skill in the art to which the tumor protein may be attached. For example, the solid support may be a test well in a microtiter plate or a nitrocellulose or other suitable membranes. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both non-covalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent (e.g., peptide or antibody of the invention) ranging from about 10 ng to about 10 µg is sufficient to immobilize an adequate amount of binding agent. Covalent attachment of binding agent to a solid support may generally be achieved by first activating the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*See, e.g.,* Pierce Immunotechnology Catalog and Handbook, 1991, at A12-A13).

One or more additional containers may enclose elements, such as reagents or buffers, to be used in the assay. Such kits may also, or alternatively, contain a detection reagent that contains a reporter group suitable for direct or indirect detection of, e.g., antibody binding. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Preferably, said detection agent is a second antibody capable of binding to a different site on the polypeptide containing a reporter group. The amount of detection reagent that remains bound to the solid support is then determined using a method appropriate for the specific reporter group.

Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent.

An alternative embodiment of the present invention relates to an above disclosed peptide/antibody which is linked to a diagnostic agent such as a chelator that is detectable upon imaging. Thus, such a peptide is useful for tumor diagnosis by imaging, e.g., magnetic resonance imaging (MRI), PET, optical imaging etc. In a preferred embodiment of the present invention, said diagnostic agent is a paramagnetic ion, radioactive ion or a fluorogenic ion. Specifically, the diagnostic agents utilized in embodiments of the invention include: chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III), erbium (III), iodine¹²³, technetium^{99m}, indium¹¹¹, rhenium¹⁸⁸, rhenium¹⁸⁶, copper⁶⁷, iodine¹³¹, yttrium⁹⁰, iodine¹²⁵, ¹²³iodine, ²¹¹astatine, ¹⁷⁷lutetium, ⁶⁸gallium and gallium⁶⁷.

Preferred tumors that can be treated/diagnosed by the compounds of the present invention are prostate tumor, thyroid carcinoma, breast cancer, cervix cancer and ENT carcinomas.

### Brief description of the drawings

Figure 1:
   **(A)** Amino acid sequence of the tumor specific peptide FROP-1
   **(B)** Results of Example 1; see Example 3 for details.
Figure 2: Assay for serum stability
   See Example 4 for details.
Figure 3: Time course of binding of FROP-1 to FRO82-2 cells
   See Example 5 for details.
Figure 4: Competition experiment; competition of ¹²⁵I-labelled FROP-1 by unlabelled FROP-1
   See Example 6 for details.
Figure 5:
   **(A)** Competition curve of FROP-1 on MCF-7 cells (unlabelled FROP-1 as competitor).
   **(B)** Time kinetics of FROP-1 binding to MCF-7 cells.
      See Example 6 for details.

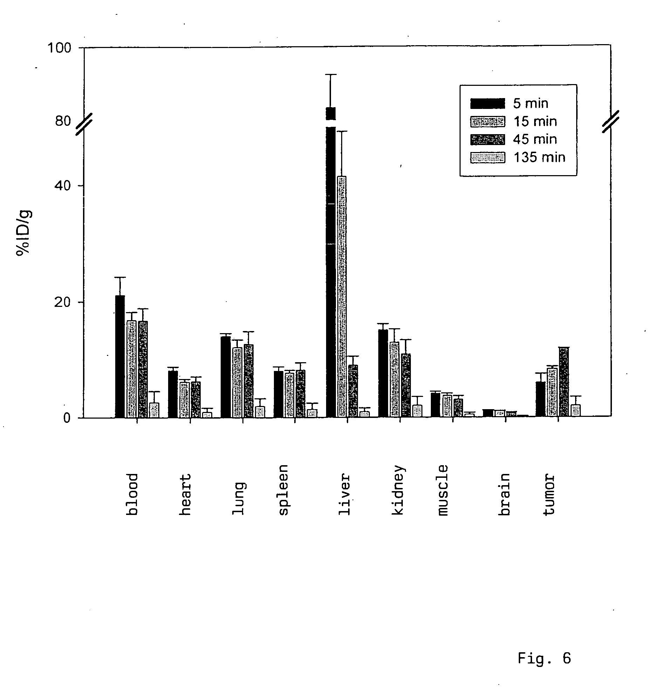

Figure 6: Accumulation of ¹³¹I-FROP-1 in various tissues of Balb/c nu/nu nude mice
   See Example 7 for details.

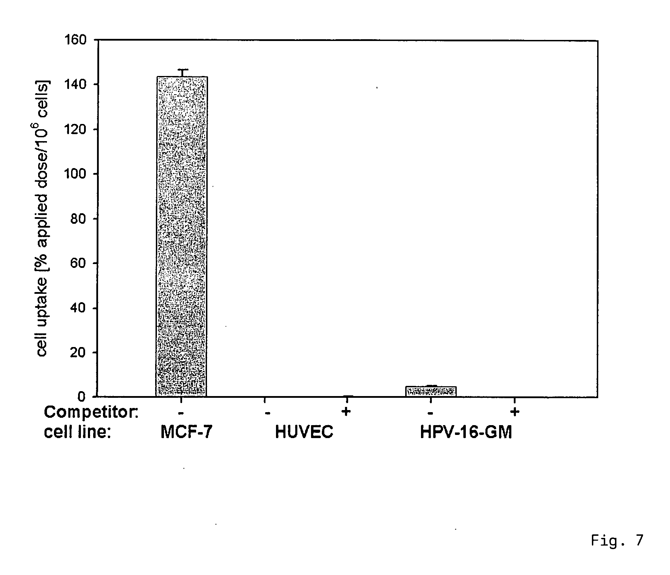

Figure 7: Binding of ¹¹¹In-FROP-1-DOTA to MCF-7 and control cells
   300,000 cells of the tumor cell line MCF7 and of the immortalized keratinocyte cell line (HPV-16-GM) and of the primary endothel cells HUVEC were each plated in 6-well plates. Prior to the experiment, the medium RPMI 1640 was replaced by 1 ml RPMI 1640 (without FCS and BSA). About 1 x 10⁶ cpm of ¹¹¹In-labelled FROP-1-DOTA per well were added and incubated for 3 hours. As a control the same experiment has been carried out in the presence of 10⁻⁴ M unlabelled FROP-1 as competitor. After incubation the cells were washed with PBS and lysed with 0.3 M NaOH. The radioactivity of lysed cells was determined with a gamma counter.
   See Example 8 for details
Figure 8:
   **(A)** Time kinetics of ¹¹¹In-FROP-1-DOTA binding to MCF-7 cells.
   **(B)** Competition curve of ¹¹¹In-FROP-1-DOTA on MCF-7 cells (unlabelled FROP-1 as competitor).
      300,000 cells of the tumor cell line MCF7 were plated in 6-well plates. Prior to the experiment, the medium RPMI 1640 was replaced by 1 ml RPMI 1640 (without FCS and BSA). (A) About 1 x 10⁶ cpm of ¹¹¹In-labelled FROP-1-DOTA per well were added and incubated for several times. As a control for unspecific binding the same experiment was carried out in 6 well plates without cells. (B) About 1 x 10⁶ cpm of ¹¹¹In-labelled FROP-1-DOTA per well were plated together with several concentrations of unlabelled FROP-1 as competitor and incubated for 3 hours.
      See Example 8 for details.
Figure 9:
   **(A)** Time kinetics of ¹¹¹In-FROP-1-DOTA binding to FRO82-2 cells.
   **(B)** Competition curve of ¹¹¹In-FROP-1-DOTA on FRO82-2 cells (unlabelled FROP-1 as competitor).
      300,000 cells of FRO82-2 were plated in 6-well plates. Prior to the experiment, the medium RPMI 1640 was replaced by 1 ml RPMI 1640 (without FCS and BSA). (A) About 1 x 10⁶ cpm of ¹¹¹In-labelled FROP-1-DOTA per well were added and incubated for several times. (B) About 1 x 10⁶ cpm of ¹¹¹In-labelled FROP-1-DOTA per well were plated together with several concentrations of unlabelled FROP-1 as competitor and incubated for 3 hours.
      See Example 8 for details.

The present invention is explained by the following examples.

### Example 1

### General methods

**(A) Mice:** Female 6-week-old BALB/c nu/nu mice were obtained from Charles River WIGA (Sulzfeld, Germany) and housed in VentiRacks. For inoculation of the tumors in nude mice a Matrigel-matrix/cell suspension (5×10⁶ cells) was injected subcutaneously into the anterior region of the mouse trunk. Tumors were grown up to a size of approximately 1.0 cm³. All animals were cared for according to the German animal guidelines.
**(B) Peptide synthesis and labeling:** The FROP-1-Peptide (EDYELMDLLAYL) was obtained by solid-phase peptide synthesis using Fmoc-chemistry. The radiolabeling was achieved by iodination using the chloramine-T method (33). The labeled peptide was purified by HPLC on a LiChrosorb RP-select B 5 µm, 250 ×x 4 mm column (Merck, Darmstadt, Germany) using TRIS/phosphate and methanol as eluents. The specific activities obtained were 90 GBq/µmol for the ¹²⁵I labeled peptide and 110 GBq/µmol and the ¹³¹I labeled peptide.
   FRO82-2 cells, DU-145, PC-3, SW1736 and FTC133 and MCF7 cells were obtained from the tumor library of the DKFZ.
**(C) Stability assay:** 100 µl ¹²⁵I-labeled peptide was added to a tube containing 1 ml human serum and incubated at 37 °C. At various time points 100 µl were removed and the proteins precipitated with 100 µl acetonitrile. The solution was centrifuged 5 min at 13,000 rpm and the cleared lysate was analyzed by HPLC.
**(D) In vitro binding assay:** 300,000 cells were seeded into 6-well plates and cultivated for 24 hours. The medium was replaced by 1 ml fresh medium (without FCS). When using the competitor, unlabeled peptide (10⁻⁴ M - 10⁻¹¹ M) was preincubated for 30 min. ¹²⁵I-labeled peptide was added to the cell culture (1-2 × 10⁶ cpm/well ) and incubated for the appropriate incubation times varying from 1 min to 4 h. The cells were washed three times with 1 ml PBS and subsequently lysed with 0.5 ml 0.3 M NaOH. Radioactivity was determined with a gamma counter and calculated as percent applied dose per 10⁶ cells. If BSA or dry milk powder were used as blocking agents, it was added to a final concentration of 1% in medium without FCS.
**(E) Organ distribution:** ¹³¹I-FROP-1 was intravenously injected into male nu/nu mice (2.8×10⁷ cpm/mouse), carrying the subcutaneously transplanted the human prostate tumors FRO82-2. At 5, 15, 45 and 135 min post injection the mice were sacrificed. The organs were removed, weighed and the radioactivity was determined using an automated NaI(Tl) well counter (CobraII, Canberra Packard, Meriden, USA). The percentage of injected dose per gram of tissue was calculated.

### Example 2

### Isolation/Identification of FROP-1

**Peptide selection:** For the selection of tumor specific peptides, 6 selection rounds, so called biopannings, were perfomed on FRO82-2 human follicular thyroid carcinoma cells. The phage display library used was the Ph.D.12 phage library from New England Biolabs, Beverly, MA, USA. Each biopanning was conducted as follows:
For the first round 5 µl of the original library (approx. 5x10¹⁰TU/ml, ~10⁹ different peptide motifs) were added to a cell culture dish with 293-cells (embryonal kidney cells) for a negative selection. After 1 h the medium was collect from the 293 cells, centrifuged for 5 min at 1500 rpm and the supernatant was transfered into a dish with FRO82-2 cells grown to 90% confluency. After 1 h the medium was removed from the DU-145 cells and the cells were washed 4 x with 10 ml of HBSS(+) + 1 % BSA and 4x with 10 ml of HBSS(-) + 1 % BSA. The cells were then detached with 4 ml of Versene for 5 min and resuspended in 16 ml RPMI 1640 medium and centrifuged 5 min at 1500 rpm. The cell pellet was resuspended in 1 ml HBSS(-) + 1 % BSA into an Eppendorf tube, centrifuged 5 min at 1500 rpm and the supernatend was removed. This wash step was repeated 3 times. Then the cell pellet was lysed with 1 % Triton. 10 µl of the lysate was used for titering. The remaining lysate was added to 50 ml of a log culture of ER2537 bacterial cells and grown 5 h for amplification. The amplified phages were precipitated with PEG (see manufacturers protocol) and the titer of the amplified phage suspension was determined. For the next biopanning 10¹¹ TU from the previous biopanning were used. 6 biopanning were performed, then single phages clones were amplified and single stranded DNA was isolated for sequencing.

### Example 3

### Uptake of ¹²⁵I-labelled FROP-1 by various cell lines

2 x 10⁵ cells of various tumor cell lines were seeded into 6-well plates. Prior to the experiment, the medium RPMI 1640 was replaced by 1 ml RPMI 1640 (without FCS and BSA). About 1 x 10⁶ cpm of ¹²⁵I-labelled FROP-1 per well were added and incubated for 1 hour. As a control, the same experiment was carried out in the presence of 10⁻⁴ unlabelled FROP-1 as a competitor. After incubation the cells were washed with PBS and lysed with 0.3 M NaOH. The radioactivity of lysed cells was determined with a gamma counter. The results are shown in Figure 1B: efficient binding of FROP-1 to MCF-7 cells (mamma carcinoma cell line), FRO82-2 cells (follicular thyroid cancer cell line), HeLa cells (cervix carcinoma cell line) and PC-3 and DU-145 (prostate carcinoma cell lines) was observed. The binding can be competed by 95%.

### Example 4

### Serum stability of FROP-1

¹²⁵I-FROP-1 was incubated in human serum at 37°C up to 120 min.

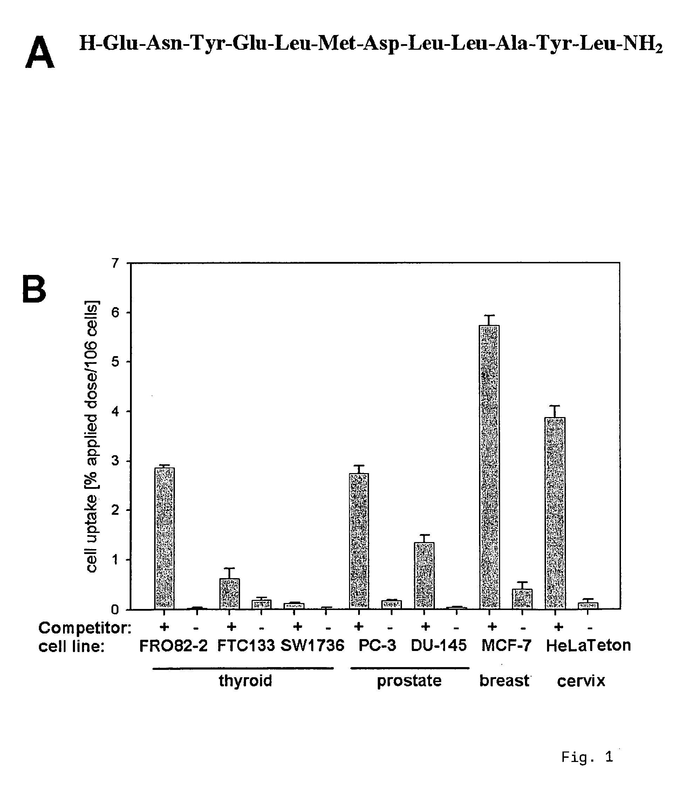

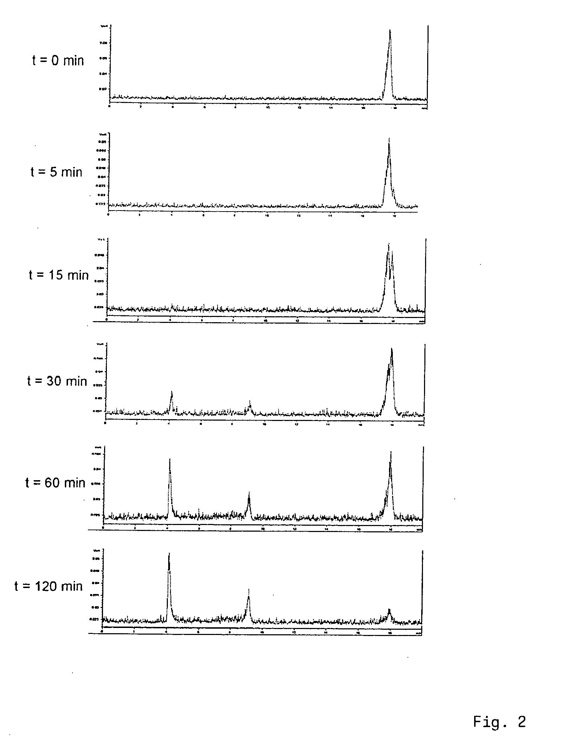

At the indicated time intervals aliquots were taken and serum proteins were precipitated with acetonitrile. Then, the samples were analyzed by HPLC. It was found that the peptide remains stable for about 15 min. After 15 min. the N-terminal amino acid glutamic acid is cleaved off and after 30 min. some additional degradaation products appear. However, even after 60 min., large amounts of full-length peptide lacking the glutamic acid were still present (Figure 2).

### Example 5

### Binding/Cell uptake of FROP-1

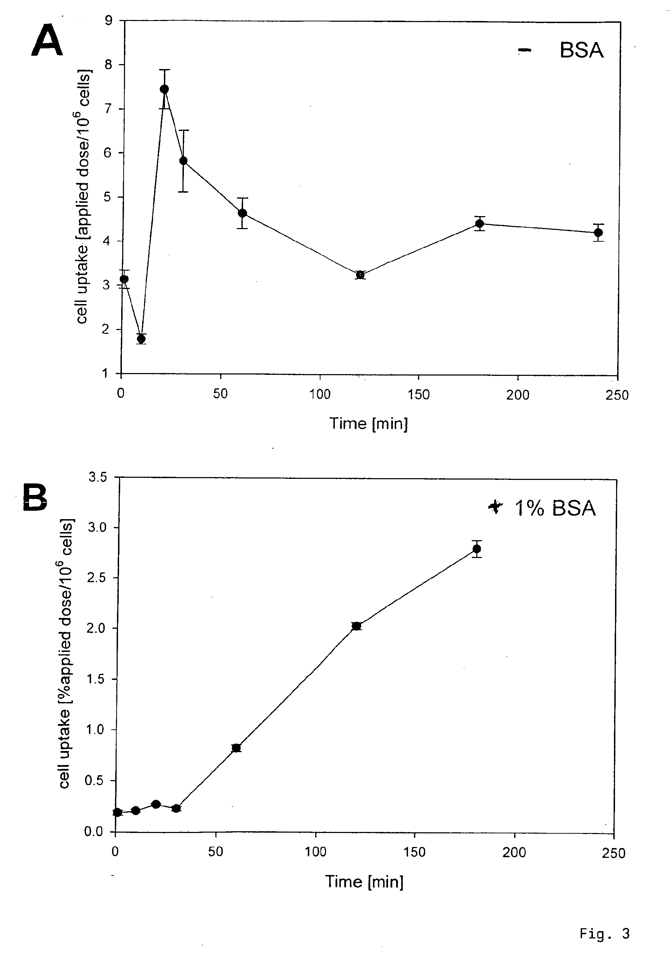

2 x 10⁵ FRO82-2 cells were seeded into 6-well plates. Prior to the experiment, the medium RPMI 1640 was replaced by 1 ml RPMI 1640 (without FCS and BSA) **(A)** or 1 ml RPMI 1640 (without FCS, but with 1% BSA). **(B)** About 1 x 10⁶ cpm of ¹²⁵I-labelled FROP-1 per well were added and incubated for various time periods. After incubation the cells were washed with PBS and lysed with 0.3 M NaOH. The radioactivity of lysed cells was determined with a gamma counter. The results are shown in Figure 3: the kinetics of peptide binding to FRO82-2- cells show an accumulation in vitro (Figure 3A), the presence of BSA does not inhibit binding but merely slows down binding which might be indicative of a depot effect of BSA (Figure 3B), i.e., BSA binds to the peptide, but peptide release is quite slow.

### Example 6

### Competition experiments

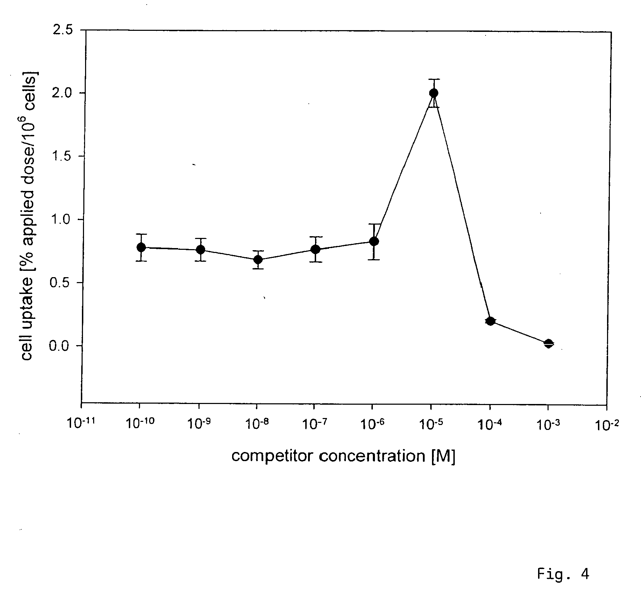

2 x 10⁵ FRO82-2 cells were seeded into 6-well plates. Prior to the experiment, the medium RPMI 1640 was replaced by 1 ml RPMI 1640 (without FCS and BSA). About 1 x 10⁶ cpm of ¹²⁵I-labelled FROP-1 per well were added together with different concentrations of competitor (unlabelled FROP-1) and incubated for 1 hour. After incubation the cells were washed with PBS and lysed with 0.3 M NaOH. The radioactivity of lysed cells was determined with a gamma counter (n = 3 per time interval, bars indicate standard deviation). The results are shown in Figure 4.

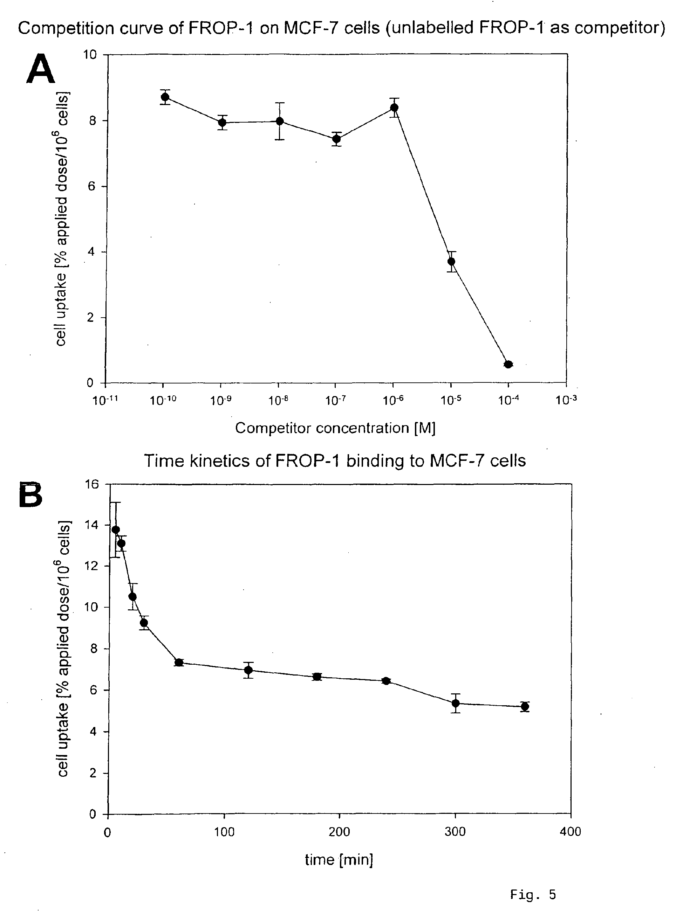

In addition, 2 x 10⁵ MCF-7 cells were seeded into 6-well plates. Prior to the experiment, the medium RPMI 1640 was replaced by 1 ml RPMI 1640 (without FCS and BSA). **(A)** About 1 x 10⁶ cpm of ¹²⁵I-labelled FROP-1 per well were added together with different concentrations of unlabelled FROP-1 as a competitor and incubated for 1 hour. **(B)** About 1 x 10⁶ cpm of ¹²⁵I-labelled FROP-1 per well were added and incubated for various time points. The results are shown in Figure 5.

The kinetics of peptide binding to MC-7 cells are indicative of a fast and high binding, which first of all decreases. However, after about 60 min. the binding is quite constant over a period of 6 hours (Figure 5B). Competition (dependent on the concentration of the unlabelled peptide) could be demonstrated for MCF-7 cells (Figure 5A) as well as for FRO-82-2 cells (Figure 4).

### Example 7

### Distribution of ¹³¹I-FROP-1 in various tissues of Balb/c nu/nu nude mice

A FRO82-2 tumor was subcutaneously implanted into Balb/c nu/nu mice. When tumor size was about 1 cm^{3 131}I-FROP-1 was applied to the animals i.v. The animals were sacrificed at the various time points and the tumor was removed from the various control tissues. ((n = 3 per time interval, bars indicate standard deviation); Fig. 6).

### Example 8

### Binding properties of the ¹¹¹In-FROP-1-DOTA in vitro

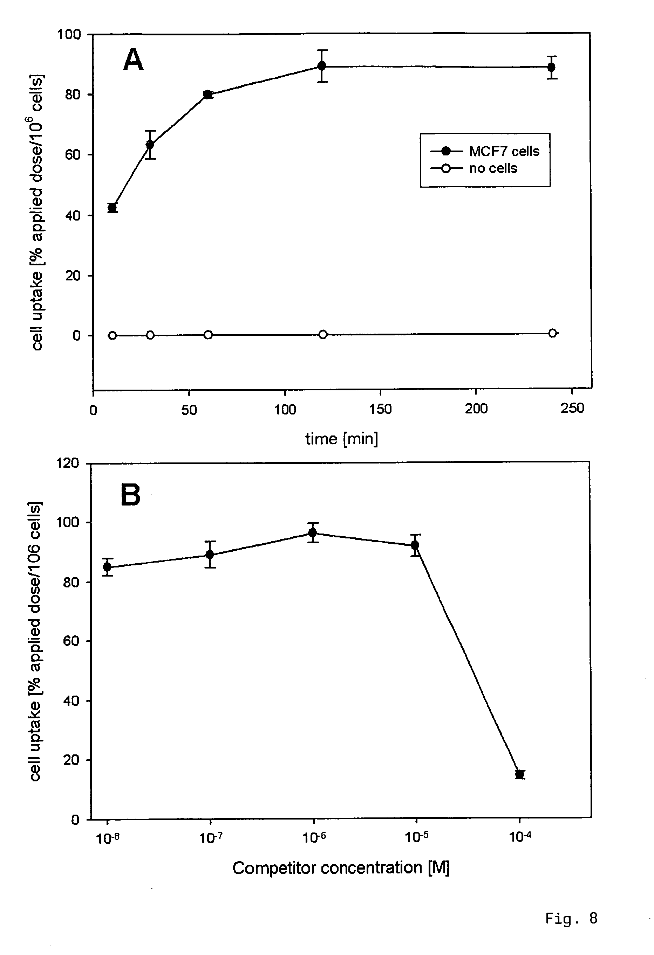

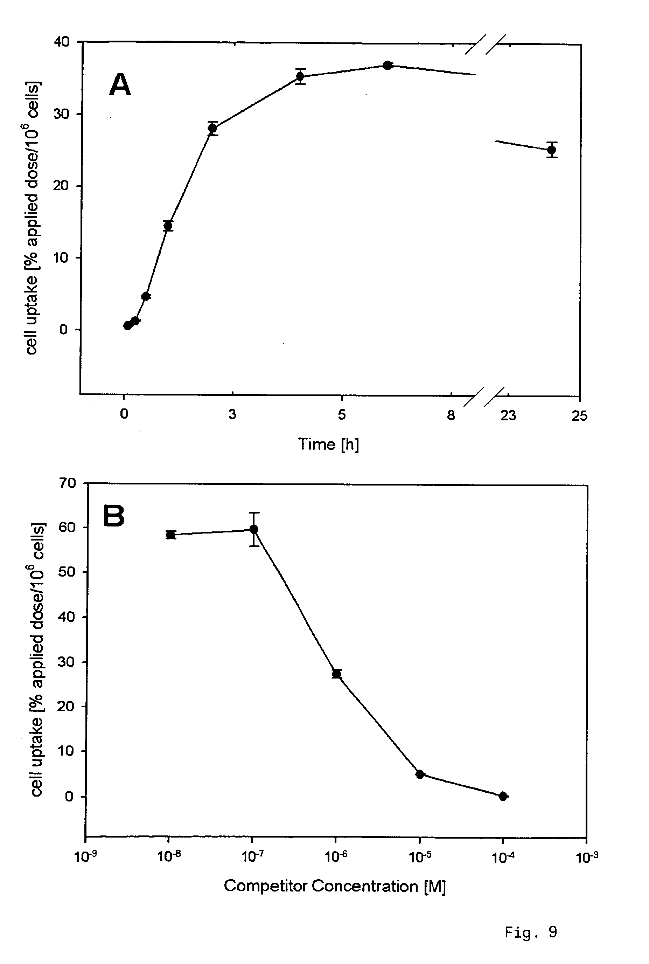

An additional lysine was added at the C-terminus of FROP-1 and was used to attach the DOTA chelator to FROP-1. The attached DOTA allows labeling of FROP-1 with various radioactive metals which is an important prerequisite for the use of FROP-1 as an imaging and therapeutic tracer. Labelling of the FROP-1-DOTA with ¹¹¹Indium showed very good binding to MCF-7 breast cancer cells while no binding to primary human endothelial cells (HUVEC) or immortalized keratinocytes (HPV-16-GM) was observed (Figure 7). This binding of ¹¹¹In-FROP-1-DOTA increased with time, reaching a plateau after 120 min on MCF-7 cells and is clearly no unspecific, as a control with empty wells showed (Figure 8A). The binding was also inhibited by the unlabelled FROP-1 as competitor at a concentration of 10⁻⁴M (Figure 8B). This strong time dependent binding of ¹¹¹In-FROP-1-DOTA was also observed for FRO82-2 cell, but to a reduced level of about 40 % cell uptake (Figure 9A). This level was reached after 4 h and decreased after 24 h only slightly. The binding was also inhibited by unlabeled FROP-1, indicating a specific binding (Figure 9B).

## Claims

1. A FROP-1 peptide capable of specifically binding to tumor cells, wherein said peptide comprises
(a) the amino acid sequence EDYELMDLLAYL;
(b) a peptide having an amino acid sequence which shows at least 60% identity to the amino acid sequence of the peptide of (a); or
(c) a peptide which is a fragment of the peptide of (a) or (b);
wherein the peptides of (b) and (c) are still capable of specifically binding to tumor cells.

2. The peptide of claim 1 which is operatively attached to a therapeutic agent capable of exerting a therapeutic effect on a tumor.

3. The peptide of claim 2, wherein said therapeutic agent is an anticellular agent, chemotherapeutic agent, a radioisotope or a cytotoxin.

4. The peptide of claim 3, wherein said cytotoxin is an A chain toxin, a ribosome inactivating protein, α-sarcin, aspergillin, restrictotin, diphtheria toxin, Pseudomonas exotoxin, a bacterial endotoxin or the lipid A moiety of a bacterial endotoxin.

5. A polynucleotide encoding the peptide of claim 1.

6. An expression vector containing the polynucleotide of claim 5.

7. An antibody or fragment thereof that specifically binds to a peptide of any one of claims 1 to 4.

8. A pharmaceutical composition containing a peptide of any one of claims 1 to 4, a polynucleotide of claim 5 or an expression vector of claim 6.

9. The peptide of claim 1 which is linked to a diagnostic agent that is detectable upon imaging.

10. The peptide of claim 9, wherein said diagnostic agent is a contrasting agent, a chromophore, a paramagnetic ion, a radioactive ion or a fluorogenic ion.

11. A diagnostic composition containing a peptide of claim 1, 9 or 10 or an antibody of claim 7.

12. Use of a peptide according to any one of claims 1 to 4, a polynucleotide of claim 5 or an expression vector of claim 6 for the preparation of a medicament for the treatment of a tumor.

13. Use of a peptide of claim 1, 9 or 10 or an antibody of claim 7 for the preparation of a diagnostic composition for tumor imaging.

14. Use according to claim 12 or 13, wherein said tumor is a prostate tumor, thyroid carcinoma, breast cancer, cervix cancer or ENT carcinoma.

15. A diagnostic kit comprising a peptide of any one of claims 1, 9 or 10 and/or an antibody of claim 7 and a detection reagent.

16. The diagnostic kit of claim 15, wherein said detection reagent comprises a reporter group.
